# FASCICULE DE BREVET EUROPEEN

(11) **EP 3 923 848 B1**
(45) Date de publication et mention de la délivrance du brevet: **12.03.2025**
(21) Numéro de dépôt: 20704303.5
(22) Date de dépôt: 13.02.2020
(51) Int. Cl.: A61B 34/30, A61B 50/13, A61B 17/58, A61B 90/11

(54) **INSTALLATION POUR LA CHIRURGIE ROBOTIQUE DU RACHIS**
INSTALLATION ZUR ROBOTISCHEN WIRBELSÄULENCHIRURGIE
INSTALLATION FOR ROBOTIC SPINE SURGERY

(30) Priorité: 14.02.2019 FR 1901512
(43) Date de publication de la demande: 22.12.2021
(73) Titulaire: S.M.A.I.O, 69800 Saint-Priest (FR)
(72) Inventeur: BUTTIN, Romain, 69800 Saint Priest (FR); DAUNE, Gautier, 69500 Bron (FR); ROUSSOULY, Pierre, 69450 Saint Cyr au Mont d'Or (FR)
(74) Mandataire: Lavoix
(86) Numéro de dépôt international: PCT/EP2020/053730
(87) Numéro de publication internationale: WO 2020/165328

(56) Documents cités:
- EP-A1- 2 893 898
- WO-A2-2006/069288
- FR-A1- 2 983 059
- US-A1- 2015 196 365
- US-A1- 2018 207 794

## Description

La présente invention concerne une installation pour la chirurgie robotique du rachis.

Le traitement chirurgical de la colonne vertébrale de patients humains peut conduire à poser des implants rachidiens. Ainsi, pour réaliser par exemple une arthrodèse d'un segment de plusieurs vertèbres, des implants sont rapportés sur ces dernières de manière à les fixer entre elles. Ces implants incluent généralement des vis dites pédiculaires, car à placer dans les pédicules des vertèbres. Les gestes chirurgicaux nécessaires à la mise en place de ces implants rachidiens, en particulier des vis pédiculaires, sont délicats à réaliser du fait des faibles dimensions des structures osseuses où ancrer les implants, de l'absence de visibilité et de la criticité des structures anatomiques avoisinantes, telles que la moelle épinière, la veine cave, l'aorte, etc.

En pratique, ces gestes chirurgicaux sont actuellement réalisés par des chirurgiens orthopédistes et neuro-orthopédistes qui, après avoir dégagé un accès postérieur aux vertèbres, utilisent sur ces dernières des outils ad hoc, notamment des outils de perçage osseux et des outils de vissage. Pour orienter leurs gestes et minimiser ainsi les risques d'endommager des structures anatomiques environnantes, les chirurgiens peuvent travailler « à main levée » en utilisant soit des repères anatomiques, soit des visées radiographiques fournies par un amplificateur de brillance, soit un système informatique de navigation peropératoire.

Depuis quelques années, les chirurgiens peuvent également être assistés par des robots chirurgicaux qui positionnent par rapport au rachis du patient à opérer un guide dans lequel ou sur lequel le chirurgien introduit ou place un outil que le chirurgien manipule lors de l'application de cet outil sur le rachis : ce guide de positionnement est par exemple un guide de perçage, qui est positionné par le robot avec précision en fonction de données peropératoires et qui est pourvu d'un trou dans lequel le chirurgien introduit un outil de perçage, étant remarqué que ce n'est pas le robot lui-même qui applique l'outil de perçage. US 2015/196365 et FR 2 983 059 en fournissent des exemples. Une telle assistance robotique présente de réels avantages pour la précision et la répétabilité des gestes du chirurgien. Elle souffre toutefois de limites liées à la présence du robot lui-même à proximité immédiate du patient opéré. En effet, le robot est typiquement installé sur une station dédiée, qui est agencée sur l'un des bords latéraux de la table opératoire où est allongé le patient et qui peut même être solidarisée à ce bord latéral de la table opératoire éventuellement de manière mobile. Dès lors, le robot et sa station occupent une place importante sur l'un des côtés latéraux de la table, en induisant nécessairement à la fois une certaine gêne pour les chirurgiens et, plus généralement, les personnels du bloc opératoire, et un risque d'interférence physique avec les autres matériels et équipements opératoires qui sont disposés autour de la table et qui sont utiles à l'intervention chirurgicale. Pour limiter quelque peu la taille de cette station, au moins une partie des moyens électroniques de commande et de contrôle du robot, dont une interface utilisée par le chirurgien, peut être déportée dans une console distante, placée hors du champ opératoire, mais cela oblige le chirurgien à des va-et-vient entre le patient et cette console et risque donc d'allonger la durée de l'intervention. Par ailleurs, la colonne vertébrale du patient opéré peut s'étendre en longueur de manière asymétrique par rapport au plan sagittal du patient, en formant des angulations plus ou moins prononcées selon une possible pathologie asymétrique du patient : lorsqu'une telle angulation fait que l'arrière de la vertèbre est tourné du côté latéral de la table opératoire, opposé à celui où se trouve la station supportant le robot, ce dernier risque de ne pas être en capacité d'amener et de positionner le guide sur cette vertèbre, sauf à déplacer la station depuis le côté latéral de la table opératoire jusqu'au côté latéral opposé de cette table, ce qui est particulièrement difficile en cours d'intervention chirurgicale, ou bien sauf à prévoir deux robots installés sur des stations respectivement situées de chaque côté de la table opératoire, ce qui est onéreux et décuple la problématique d'encombrement évoquée plus haut.

Dans un domaine différent de celui du traitement chirurgical du rachis, EP 2 893 898 divulgue un système médical robotisé qui est prévu pour la manipulation de cathéters et de matériels associés. Ce système médical robotisé comporte une table opératoire sur laquelle un patient peut s'allonger. Il comporte également une arche constituée de deux montants verticaux et d'une poutre horizontale, qui relie les montants au-dessus de la table opératoire et qui supporte des têtes robotisées de manipulation de cathéters. Une caractéristique essentielle de ce système médical robotisé est que les montants verticaux sont reliés mécaniquement aux côtés latéraux respectifs de la table opératoire, afin de maintenir en position et guider en translation l'arche par rapport à la table opératoire, ce qui rend le système encombrant et inadapté à la présence des personnels et des équipements que nécessite la chirurgie du rachis.

US 2018/0207794 divulgue, quant à lui, une installation pour la chirurgie robotique du rachis, qui répond au préambule de la revendication 1 annexée.

Le but de la présente invention est de proposer une nouvelle installation de chirurgie robotique du rachis, dont l'agencement soit plus pratique et plus performant.

A cet effet, l'invention a pour objet une installation pour la chirurgie robotique du rachis, telle que définie à la revendication 1.

Une des idées à la base de l'invention est de positionner le robot chirurgical non pas latéralement au patient allongé à opérer, mais au-dessus et en alignement avec ce patient. Pour ce faire, l'invention prévoit que le robot chirurgical puisse s'étendre à l'aplomb du rachis du patient allongé, en étant supporté par une plateforme qui surplombe le patient et qui s'étend transversalement à la colonne vertébrale de ce dernier. Cette plateforme est en appui sur deux montants opposés, qui sont prévus de part et d'autre du patient allongé et qui s'étendent chacun depuis la plateforme jusqu'au sol. Ainsi, cette plateforme et ses montants forment conjointement une table-pont qui enjambe le patient : les personnels du bloc opératoire et les autres matériels et équipements opératoires ne sont pas gênés par le robot et par la partie de la plateforme, qui est située en surplomb du patient allongé et qui supporte le robot, tandis que le reste de la table-pont peut être dimensionné pour ne présenter qu'un faible encombrement et peut même avantageusement intégrer des aménagements pratiques, tels que des zones utilisées par les personnels du bloc pour déposer ou ranger des instrumentations chirurgicales, et/ou tels qu'une armoire où sont logés les systèmes électroniques, notamment des contrôleurs, utiles à la commande et au contrôle de l'installation, en particulier du robot. De plus, grâce à son agencement à l'aplomb du rachis du patient allongé opéré, le robot est positionné au plus près des vertèbres à opérer quel que soit le patient, ce qui facilite l'approche des vertèbres par le robot sans que ce dernier n'ait à s'étendre selon des cinématiques longues et complexes, évitant ainsi de devoir utiliser de « gros » robots ayant des champs d'action très étendus, et le robot peut travailler dans les mêmes conditions quelle que soit l'éventuelle asymétrie gauche-droite du rachis opéré.

L'invention peut être mise en œuvre avec un robot d'assistance aux actes chirurgicaux, tel qu'évoqué plus haut. Cela dit, l'invention trouve une application particulièrement avantageuse pour le cas où le robot permet de réaliser lui-même un acte chirurgical, tel que le perçage des pédicules d'une vertèbre du patient opéré et/ou l'introduction dans ces pédicules d'une vis pédiculaire.

Des caractéristiques additionnelles avantageuses de l'installation conforme à l'invention sont spécifiées aux autres revendications.

L'invention sera mieux comprise à la lecture de la description qui va suivre, données uniquement à titre d'exemple et faite en se référant aux dessins sur lesquels :
[Fig 1] la figure 1 est une vue en perspective d'une installation conforme à l'invention ;
[Fig 2] la figure 2 est une vue en élévation selon la flèche II de la figure 1 ;
[Fig 3] la figure 3 est une coupe partielle schématique selon la ligne III-III de la figure 2 ; et
[Fig 4] la figure 4 est une vue en élévation selon la flèche IV de la figure 1, illustrant l'installation lors d'un temps opératoire différent de celui montré à la figure 1.

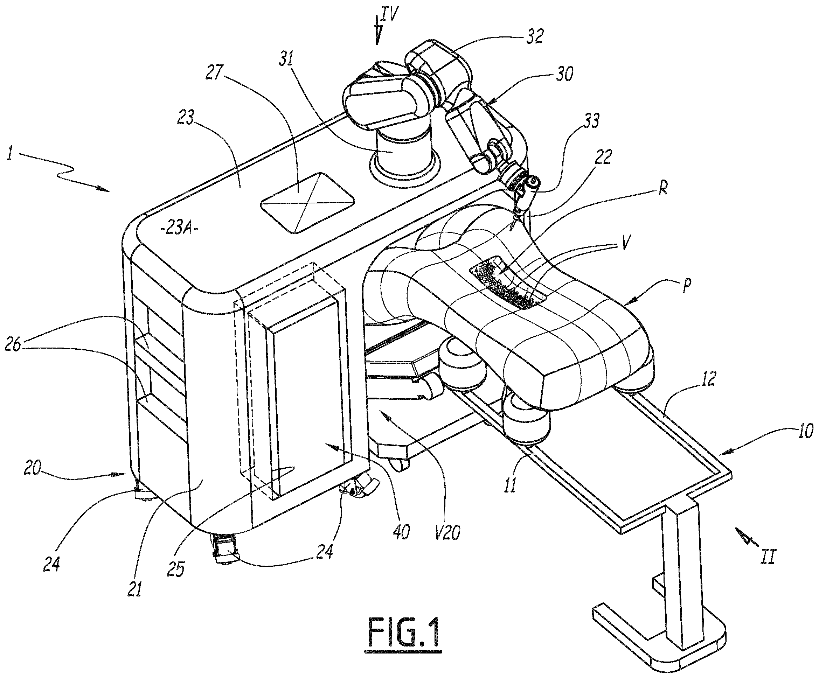

Sur les figures 1 à 4 est représentée une installation 1 permettant de pratiquer des traitements chirurgicaux du rachis des êtres humains.

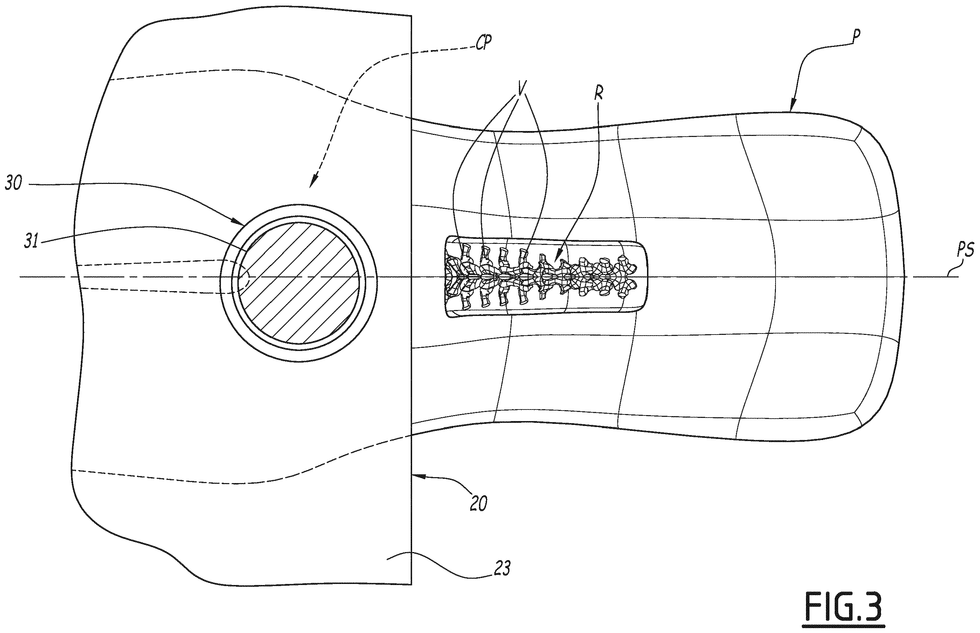

Comme bien visible sur les figures 1, 2 et 4, l'installation 1 comporte une table opératoire 10 sur laquelle un patient P à opérer peut être allongé. Lors d'une intervention chirurgicale utilisant l'installation 1, le patient P est allongé sur le ventre sur la table opératoire 10 : l'arrière du patient est ainsi tourné vers le haut et l'accès aux vertèbres V du patient P est opéré par voie postérieure, comme montré schématiquement sur les figures 1 et 3. On notera que sur les figures, le patient P n'est dessiné que de manière schématique et partielle, seul le tronc et la partie haute des jambes du patient étant représentés.

La forme de réalisation de la table opératoire 10 n'est pas limitative de l'invention, du moment que cette table opératoire permet d'y allonger le patient P sur le ventre. Quelle que soit sa forme de réalisation, la table opératoire 10 présente deux côtés latéraux opposés 11 et 12, qui s'étendent de part et d'autre et parallèlement au plan sagittal PS du patient allongé P.

L'installation 1 comporte également une table-pont 20 qui comprend principalement, voire exclusivement, des montants 21 et 22 et une plateforme 23.

Les montants 21 et 22 s'élèvent depuis le sol, de manière globalement parallèle l'un à l'autre, en étant distants l'un de l'autre de manière à former un espace libre entre eux. L'écartement entre les montants 21 et 22 est supérieur à la distance séparant l'un de l'autre les côtés latéraux 11 et 12 de la table opératoire 10. La plateforme 23 repose sur les montants 21 et 22, en reliant l'une à l'autre les extrémités respectives des montants 21 et 22, opposées au sol.

La plateforme 23 est ainsi située en hauteur par rapport au sol et ferme vers le haut un volume libre V20 entre les montants 21 et 22. En pratique, la plateforme 23 est située à une hauteur, par rapport au sol, qui est suffisamment grande pour que, lorsque l'installation 1 est en service, la plateforme 23 surplombe le patient P allongé sur la table opératoire 10 alors que celle-ci s'étend en longueur au travers du volume libre V20, comme bien visible sur les figures. Autrement dit, lorsque l'installation 1 est en service, les montants 21 et 22 sont disposés de part et d'autre des côtés latéraux 11 et 12 de la table opératoire 10 et s'élèvent ainsi de part et d'autre du patient P allongé sur la table opératoire 10, tandis que, dans le même temps, la plateforme 23 est située au-dessus de ce patient et s'étend entre les montants 21 et 22 suivant une direction qui est transversale au plan sagittal PS du patient P.

A leur base, c'est-à-dire à leur extrémité tournée vers le sol, les montants 21 et 22 sont avantageusement pourvus de roulettes avec frein 24 permettant, alternativement, de déplacer la table-pont 20 sur le sol par roulement des roulettes et de fixer de manière stable une position choisie de la table-pont 20 le long du patient P allongé sur la table opératoire 10 par action du frein des roulettes. Bien entendu, les roulettes avec frein 24 ne sont qu'un exemple de réalisation possible pour des éléments de déplacement et de stabilisation par rapport au sol, qui équipent les montants 21 et 22 et qui, en service, permettent alternativement de changer la position de la table-pont 20 le long du patient allongé par déplacement sur le sol, par exemple par roulement ou par glissement, et d'immobiliser ensuite la table-pont 20 de manière stable sur le sol, par exemple par blocage en mouvement ou par appui pressant voire soulevant par rapport au sol.

La table-pont 20, en particulier ses montants 21 et 22, est séparée de la table opératoire 10, dans le sens où aucune liaison mécanique n'est prévue entre la table-pont et la table opératoire.

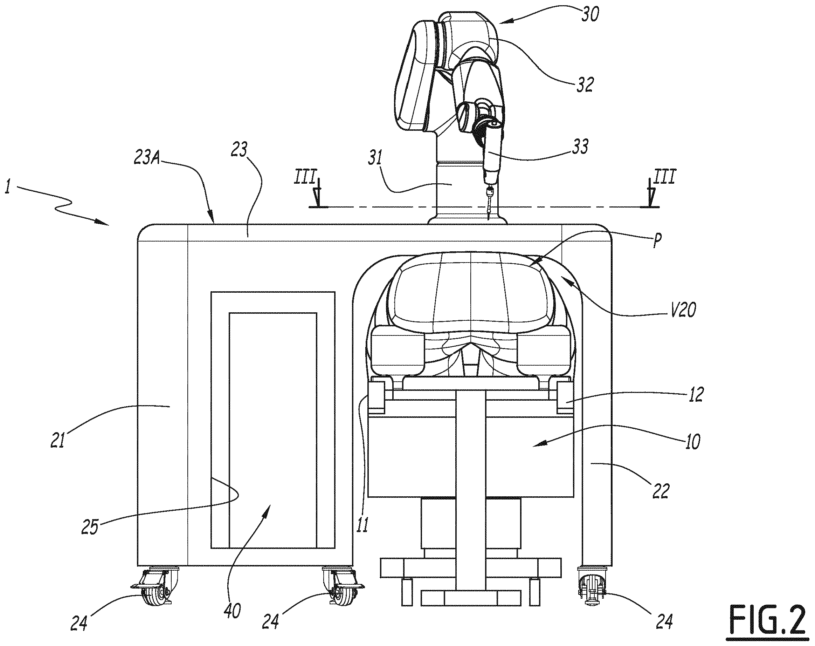

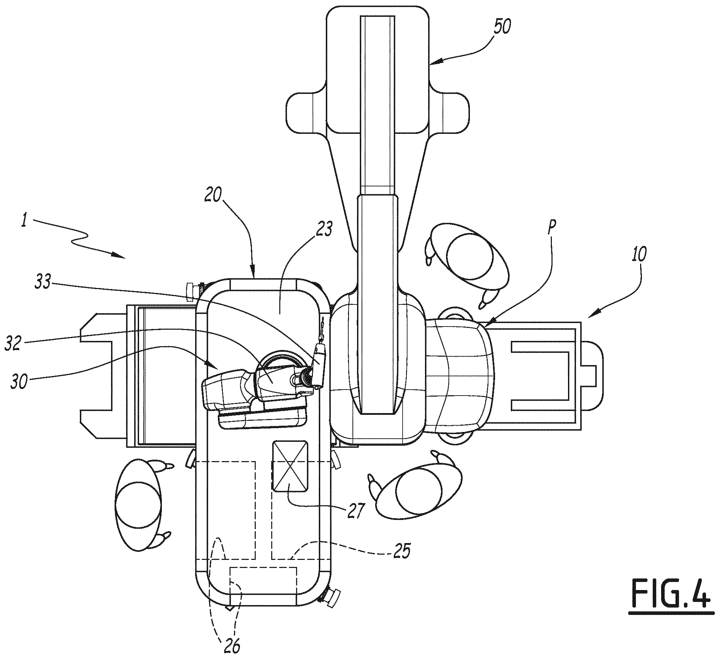

Pour des raisons qui apparaîtront un peu plus loin, le montant 21 présente avantageusement des aménagements optionnels qui font que ce montant 21 n'est pas identique au montant 22 mais présente notamment une dimension, dans la direction selon laquelle la plateforme 24 relie les montants 21 et 22, qui est plus grande que celle du montant 22. Ces aménagements optionnels incluent une armoire 25, montrée sur les figures 1 et 2, et une ou plusieurs niches 26, montrées sur les figures 1 et 4. L'armoire 25 et les niches 26 sont intégrées au montant 21, en débouchant chacune sur au moins l'une des faces du montant 21, autres que celle délimitant le volume libre V20.

Egalement pour des raisons qui apparaîtront plus loin, la plateforme 23 intègre, sur sa face supérieure 23A, c'est-à-dire sa face opposée aux montants 21 et 22, une zone fonctionnelle 27 qui est repérée par une croix sur les figures 1 et 4. Dans l'exemple de réalisation considéré ici, cette zone fonctionnelle 27 est au moins en partie située à l'aplomb du montant 21.

L'installation 1 comporte en outre un robot chirurgical 30. Dans l'exemple de réalisation considéré ici, ce robot 30 comporte un pied 31, ainsi qu'un bras robotisé 32 qui s'étend depuis le pied 31 et incluant à cet effet des tronçons successifs, à savoir un premier tronçon porté de manière mobile par le pied 31, un deuxième tronçon porté de manière mobile par le premier tronçon, et ainsi de suite. Le robot 30 comporte également un organe effecteur 33, qui termine le bras robotisé 32 à l'opposé du pied 31 et qui est porté par le dernier tronçon du bras robotisé 32. Les spécificités de structure et de mobilité du bras robotisé 32 ne sont pas limitatives de l'invention : ainsi, chacun des tronçons du bras robotisé 32 peut ainsi être mobile en rotation ou en translation par rapport au tronçon ou au pied 31 le portant. Plus globalement, la nature du robot 30 n'est pas limitative du moment que ce robot est capable de participer à une chirurgie postérieure du rachis.

Suivant un mode de réalisation préférentiel, l'organe effecteur 33 du robot 30 est adapté pour pratiquer un acte chirurgical sur le patient P, c'est-à-dire pour appliquer une énergie entamant le rachis R, cette énergie pouvant être de nature mécanique, radiative, laser, etc. Ainsi, l'organe effecteur 33 est alors prévu pour agir matériellement sur le rachis R, notamment en perçant ou coupant les vertèbres et/ou en y implantant, notamment par vissage, des dispositifs médicaux à rapporter. L'organe effecteur 33 est par exemple adapté pour percer le pédicule des vertèbres V du patient P ou pour introduire une vis pédiculaire dans les pédicules vertébraux du patient. Plus généralement, quelles que soient les spécificités de l'organe effecteur 33, le robot 30 est ainsi avantageusement configuré pour, en service, réaliser un acte chirurgical sur le rachis R du patient P allongé sur la table opératoire 10.

Quelle que soit sa forme de réalisation, le robot 30 est supporté par la plateforme 23 de la table-pont 20, en reposant et en s'étendant depuis la face supérieure 23A de la plateforme 23, comme bien visible sur les figures 1 à 4. Plus précisément, le robot est ainsi supporté par la plateforme 23 de manière que sa jonction avec cette dernière est située, à la fois, à l'aplomb du volume libre V20, autrement dit verticalement au-dessus de l'écartement libre entre les montants 21 et 22, et sensiblement à même distance des montants 21 et 22 dans la direction selon laquelle la plateforme 24 relie ces montants 21 et 22. Lorsque l'installation 1 est en service, le robot 30 peut ainsi s'étendre à l'aplomb du rachis R du patient P allongé sur la table opératoire 10, comme illustré par les figures 1 et 2. Cet agencement du robot 30 sur la plateforme 23 permet au robot 30 d'être positionné au plus près de la région opérée du patient P et de s'étendre depuis la plateforme 23 à la fois le long et verticalement au-dessus du rachis R du patient, en pouvant s'aligner sensiblement dans le plan sagittal PS du patient allongé : le robot 30 travaille alors de façon particulièrement performante, et ce avec un même niveau de performance sur les côtés gauche et droit du rachis R, en s'adaptant ainsi à des éventuelles pathologies induisant une asymétrie gauche-droite des vertèbres V.

Suivant une forme préférentielle de cet agencement, qui est mise en œuvre dans l'exemple considéré sur les figures et qui est plus particulièrement montrée à la figure 3, le pied 31 du robot 30 est solidarisé à la plateforme 23 de manière à, lorsque l'installation 1 est en service, être sensiblement centré sur le plan sagittal PS du patient P allongé sur la table opératoire 10. En pratique, la solidarisation du pied 31 sur la plateforme 23 peut être prévue fixe, comme dans l'exemple de réalisation considéré sur les figures. Cela étant, à titre de variante non représentée, le pied 31 peut être solidarisé à la plateforme 23 de manière mobile afin de pouvoir ajuster leur positionnement relatif, cette mobilité pouvant être en translation selon une direction qui, en service, est parallèle ou perpendiculaire au plan sagittal PS du patient allongé P et/ou cette mobilité pouvant être en rotation autour d'un axe perpendiculaire au plan sagittal PS du patient allongé P.

Le robot 30 est avantageusement prévu pour s'étendre sur un champ d'action, c'est-à-dire dans une région d'espace atteignable par son organe effecteur 33 moyennant le déplacement de son bras robotisé 32 par rapport au pied 31 et, par-là, par rapport à la plateforme 23, qui couvre plusieurs vertèbres du patient allongé P. Ainsi, lorsque l'installation 1 est en service et que la table-pont 20 occupe une position fixe le long du patient P allongé sur la table opératoire 10, le robot 30 a, dans son champ d'action, plusieurs vertèbres V du patient allongé, le robot 30 étant ainsi prévu pour que son champ d'action couvre de préférence entre trois et huit vertèbres, notamment cinq vertèbres. De cette façon, l'organe effecteur 33 du robot 30 peut agir directement sur chacune de ces vertèbres V sans avoir à modifier le positionnement relatif entre la table-pont 20 et le patient allongé P.

En pratique, un positionnement préféré entre la table-pont 20 et le patient allongé P, qui résulte d'une conception appropriée de la table-pont et qui est mis en œuvre dans l'exemple illustré sur les figures, est que, en service, la table-pont 20 positionne le pied 31 du robot 30 verticalement au-dessus de la ceinture pelvienne CP du patient P allongé sur la table opératoire 10, cette ceinture pelvienne s'étendant, suivant l'axe du patient P, de la jonction du sacrum avec la colonne lombaire du patient, jusqu'à l'articulation des hanches du patient, comme bien visible sur la figure 3. De cette façon, le robot 30 peut, en service, intervenir de manière performante et efficace sur les vertèbres qui sont fréquemment traitées par chirurgie postérieure, à savoir les vertèbres sacrées et les vertèbres lombaires du patient allongé P, ainsi qu'au moins certaines des vertèbres dorsales du patient, en particulier ses trois dernières vertèbres dorsales. Ainsi, selon la ou les vertèbres V à traiter pour un patient donné et selon l'étendue du champ d'action du robot 30, le pied 31 du robot peut demeurer situé verticalement au-dessus de la ceinture pelvienne CP du patient allongé P pendant toute la durée d'actionnement du robot 30 au cours de l'intervention chirurgicale, notamment sans avoir à modifier le positionnement relatif entre la table-pont 20 et le patient allongé P. Bien entendu, si besoin, la table-pont 20 peut, de manière peropératoire, être déplacée le long du patient allongé P, notamment en direction de la tête de ce dernier, si bien que le pied 31 du robot peut se retrouver verticalement au-dessus du sacrum du patient allongé et donc rester dans les limites de la ceinture pelvienne CP, voire se retrouver verticalement au-dessus de la région lombaire ou même dorsale du rachis R et donc au-delà de la ceinture pelvienne CP du patient allongé.

Le champ d'action du robot 30 couvre également la zone fonctionnelle 27 de la plateforme 23. De cette façon, l'organe effecteur 33 du robot 30 peut atteindre cette zone fonctionnelle 27, moyennant un mouvement approprié du bras robotisé 32. Dès lors, en disposant des implants rachidiens sur cette zone fonctionnelle 27, ces implants se trouvent présentés à l'organe effecteur 33 de manière que ce dernier peut les saisir ou, plus généralement, les charger depuis cette zone de présentation 27 avant que le robot 30 ne les entraîne jusqu'au rachis R du patient allongé P.

L'installation 1 comporte également une unité électronique 40 qui n'est représentée que de manière schématique sur les figures 1 et 2. Cette unité électronique 40 inclut divers matériels électroniques, électriques et informatiques, permettant de commander l'installation 1. En particulier, l'unité électronique 40 est adaptée pour commander le robot 30 : à cet effet, l'unité électronique 40 inclut par exemple un premier contrôleur pour commander le déplacement du robot 30 par rapport à la plateforme 23, en particulier pour commander les mouvements du bras robotisé 32, et un second contrôleur pour commander l'actionnement de l'organe effecteur 33. L'unité électronique 40 peut, en option, inclure également un ordinateur exécutant, entre autres, un logiciel de planification opératoire. Quelles que soient les spécificités matérielles et fonctionnelles de l'unité électronique 40, cette unité électronique 40 est avantageusement logée à l'intérieur de l'armoire 25 de la table-pont 20, en optimisant ainsi l'encombrement et l'ergonomie de l'installation 1.

Avantageusement, l'unité électronique 40 est associée à une interface pour interagir avec un chirurgien, cette interface étant reliée au reste de l'unité électronique 40, notamment aux contrôleurs et à l'ordinateur précité : grâce à cette interface, le chirurgien commande et contrôle l'installation 1, en particulier le robot 30. En pratique, cette interface, qui n'est pas représentée sur les figures, peut être supportée par la table-pont 20 à une hauteur appropriée pour le chirurgien.

Lors du fonctionnement de l'installation 1, le robot 30 est commandé par le chirurgien, en suivant des consignes saisies par ce dernier dans l'unité électronique 40 via l'interface précitée. Avant que le robot 30 n'exécute les consignes données par le chirurgien, les roulettes avec frein 24 permettent d'ajuster facilement la position de la table-pont 20 et, par-là, du robot 30, le long du patient P allongé sur la table opératoire 10 et permettent, une fois cette position atteinte, de figer cette dernière de manière stable. Le robot 30 exécute alors les consignes données sans que sa présence, ni les mouvements qu'il met en œuvre ne gênent le chirurgien, ni, plus généralement, les personnels du bloc opératoire, grâce à l'agencement en hauteur du robot 30, sur la plateforme 23. Comme expliqué plus haut, les opérations mises en œuvre par le robot 30 sont réalisées de façon optimale, dans le sens où le robot s'étend directement à l'aplomb du rachis R du patient allongé P, si nécessaire en s'adaptant à des asymétries aussi bien gauche que droite de ce rachis R.

Lors de l'intervention chirurgicale, l'agencement de l'installation 1 permet au robot 30 de ne pas interférer avec d'autres matériels et équipements du bloc opératoire, comme illustré à la figure 4. Sur cette figure 4, un imageur 50, typiquement un amplificateur de brillance, est utilisé sur le patient P allongé sur la table opératoire 10, en s'étendant en partie au-dessus du rachis R du patient P : la table-pont 20 ne gêne pas l'utilisation de cet imageur 50 et le robot 30 n'entre pas en contact avec l'imageur 50, si besoin par déplacement du robot par rapport à la plateforme 23 en le repliant, comme illustré par la figure 4. Plus généralement, on comprend que la table-pont 20 et le robot 30 permettent de ne pas déranger les procédures conventionnelles des chirurgiens, notamment la mise en place et l'installation d'autres matériels et équipements opératoires, utiles à la bonne exécution de la chirurgie rachidienne.

Egalement lors de l'intervention chirurgicale, l'instrumentation chirurgicale destinée à être manipulée à la main par le chirurgien est avantageusement mise à disposition dans les niches 26 de la table-pont 20 et/ou sur la face supérieure 23A de la plateforme 23 : cette instrumentation chirurgicale est par exemple déposée ou rangée dans les niches 26 et/ou sur la plateforme 23 par un infirmier du bloc opératoire, avant que le chirurgien puisse s'en saisir facilement lorsqu'il en a besoin. On comprend ainsi que l'utilisation de matériels opératoires annexes, tels qu'une servante mobile, peut ainsi être évitée. Bien entendu, les niches 26 montrées sur les figures ne sont que des exemples d'une ou de zones de mise à disposition pour de l'instrumentation chirurgicale, qui sont avantageusement intégrées à l'un et/ou l'autre des montants 21 et 22 de la table-pont 20, en plus d'une ou plusieurs autres zones fonctionnellement similaires, intégrées sur la face supérieure 23A de la plateforme 23 de la table-pont.

Par ailleurs, divers aménagements et variantes à l'installation 1 décrite jusqu'ici sont envisageables. A titre d'exemples :
- plutôt que le robot 30 soit configuré pour, en service, réaliser un acte chirurgical sur le patient opéré, ce robot peut être configuré pour uniquement assister un tel acte chirurgical, ce dernier étant appliqué par un chirurgien sur le patient P allongé sur la table d'opération 10 ; dans ce cas, l'organe effecteur 33 est par exemple un guide, tel qu'un guide de perçage, comme évoqué dans la partie introductive du présent document ; et/ou
- la structure et les matériaux de la table-pont 20 sont indifférents, du moment que cette table-pont 20 puisse être utilisée dans un bloc opératoire et qu'elle supporte sans risque de rupture ou d'instabilité le robot 30.

## Revendications

1. Installation (1) pour la chirurgie robotique du rachis, comportant :
- une table opératoire (10), qui est adaptée pour y allonger un patient (P),
- un robot (30) de chirurgie postérieure du rachis, le robot étant configuré pour, en service, réaliser un acte chirurgical sur le patient allongé sur la table opératoire, et
- une table-pont (20) comprenant :
- deux montants (21, 22) qui sont adaptés pour, lorsque l'installation est en service, être séparés de la table opératoire tout en étant disposés et en s'élevant, depuis le sol, de part et d'autre du patient allongé sur la table opératoire, et
- une plateforme (23) de support pour le robot (30), laquelle plateforme repose sur les montants de manière à, en service, surplomber le patient allongé sur la table opératoire et laquelle plateforme est adaptée pour, en service, supporter le robot s'étendant à l'aplomb du rachis (R) du patient allongé sur la table opératoire
**caractérisée en ce que** la plateforme (23) intègre une zone de présentation (27) pour des implants rachidiens, cette zone de présentation étant couverte par un champ d'action sur lequel le robot (30) est prévu pour s'étendre.

2. Installation suivant la revendication 1, dans laquelle le robot (30) est prévu pour s'étendre sur un champ d'action qui, lorsque l'installation (1) est en service et que la table-pont (20) occupe une position fixe le long du patient (P) allongé sur la table opératoire (10), couvre plusieurs vertèbres (V) du patient, notamment entre trois et huit vertèbres du patient.

3. Installation suivant l'une quelconque des revendications précédentes, dans laquelle le robot (30) comprend :
- un pied (31) qui est solidarisé à la plateforme (23) de la table-pont (20) de manière à, en service, être sensiblement centré sur le plan sagittal (PS) du patient (P) allongé sur la table opératoire (10), et
- un bras robotisé (32), qui s'étend depuis le pied (31), en étant, en service, positionné à l'aplomb du rachis (R) du patient (P), et qui se termine par un organe effecteur de chirurgie rachidienne (33).

4. Installation suivant la revendication 3, dans laquelle la table-pont (2) est conçue pour, en service, positionner le pied (31) du robot (30) verticalement au-dessus de la ceinture pelvienne (CP) du patient (P) allongé sur la table opératoire (10).

5. Installation suivant l'une quelconque des revendications précédentes, dans laquelle le robot (30) est configuré pour, en service, percer le pédicule d'une vertèbre (V) du patient allongé sur la table opératoire (10).

6. Installation suivant l'une quelconque des revendications 1 à 4, dans laquelle le robot (30) est configuré pour, en service, introduire une vis dans le pédicule d'une vertèbre du patient allongé sur la table opératoire (10).

7. Installation suivant l'une quelconque des revendications précédentes dans laquelle le robot (30) est configuré pour, en service, assister un acte chirurgical appliqué par un chirurgien au patient (P) allongé sur la table opératoire (10), notamment pour guider un outil de perçage appliqué par le chirurgien sur le pédicule d'une vertèbre du patient.

8. Installation suivant l'une quelconque des revendications précédentes, dans laquelle les montants (21, 22) sont pourvus d'éléments de déplacement et de stabilisation (24) par rapport au sol, ces éléments de déplacement et de stabilisation étant, en service, adaptés pour, alternativement, changer la position de la table-pont (20) le long du patient (P) allongé sur la table opératoire (10) par déplacement sur le sol et immobiliser la table-pont de manière stable sur le sol.

9. Installation suivant la revendication 8, dans laquelle les éléments de déplacement et de stabilisation sont des roulettes avec frein (24).

10. Installation suivant l'une quelconque des revendications précédentes, dans laquelle l'un des montants (21, 22) intègre une armoire (25) à l'intérieur de laquelle est logée une unité électronique (40) adaptée pour commander le robot (30).

11. Installation suivant l'une quelconque des revendications précédentes, dans laquelle la table-pont (20) intègre, sur une face supérieure (23A) de sa plateforme (23), au moins une zone de mise à disposition (26) pour de l'instrumentation chirurgicale.

12. Installation suivant l'une quelconque des revendications précédentes, dans laquelle la table-pont (20) intègre, dans au moins l'un de ses montants (11, 12), au moins une zone de mise à disposition (26) pour de l'instrumentation chirurgicale.

## Patentansprüche

1. Installation (1) zur robotischen Wirbelsäulenchirurgie, umfassend:
- einen Operationstisch (10), der ausgelegt ist, um einen Patienten (P) darauf zu legen,
- einen Roboter (30) zur hinteren Chirurgie der Wirbelsäule, wobei der Roboter konfiguriert ist, um im Betrieb einen chirurgischen Eingriff an dem Pateinten durchzuführen, der auf dem Operationstisch liegt, und
- einen Brückentisch (20), umfassend:
- zwei Säulen (21, 22), die ausgelegt sind, um, wenn die Installation im Betrieb ist, vom Operationstisch getrennt zu sein und gelichzeitig und in der Erhöhung vom Boden auf beiden Seiten des Patienten angeordnet zu sein, der auf dem Operationstisch liegt, und
- eine Stützplattform (23) für den Roboter (30), wobei die Plattform auf den Säulen derart aufliegt, dass sie im Betrieb den Patienten überragt, der auf dem Operationstisch liegt, und wobei die Plattform ausgelegt ist, um, im Betrieb, den Roboter zu stützen, der sich direkt über der Wirbelsäule (R) des Patienten, der auf dem Operationstisch liegt, erstreckt,
**dadurch gekennzeichnet, dass** die Plattform (23) einen Präsentationsbereich (27) für Wirbelsäulenimplantate umfasst, wobei dieser Präsentationsbereich von einem Betätigungsfeld bedeckt ist, auf dem der Roboter (30) sich erstrecken soll.

2. Installation nach Anspruch 1 wobei vorgesehen ist, dass sich der Roboter (30) auf einem Betätigungsfeld erstreckt, das, wenn die Installation (1) im Betrieb ist und der Brückentisch (20) eine feste Position entlang des Patienten (P) einnimmt, der auf dem Operationstisch (10) liegt, mehrere Wirbel (V) des Patienten abdeckt, insbesondere zwischen drei und acht Wirbel des Patienten.

3. Installation nach einem der vorhergehenden Ansprüche, wobei der Roboter (30) Folgendes umfasst:
- einen Fuß (31), der fest mit der Plattform (23) des Brückentischs (20) verbunden ist, so dass er im Betrieb im Wesentlichen auf der Sagittalebene (PS) des Patienten (P) zentriert ist, der auf dem Operationstisch (10) liegt, und
- einen automatisierten Arm (32), der sich vom Fuß (31) erstreckt, indem er im Betrieb direkt über der Wirbelsäule (R) des Patienten (P) positioniert ist, und der in einem Effektororgan der Wirbelsäulenchirurgie (33) endet.

4. Installation nach Anspruch 3, wobei der Brückentisch (2) entworfen ist, um im Betrieb den Fuß (31) des Roboters (30) vertikal über den Beckengürtel (CP) des Patienten (P) zu positionieren, der auf dem Operationstisch (10) liegt.

5. Installation nach einem der vorhergehenden Ansprüche, wobei der Roboter (30) konfiguriert ist, um im Betrieb das Pedikel eines Wirbels (V) des Patienten, der auf dem Operationstisch (10) liegt, zu durchbrechen.

6. Installation nach einem der Ansprüche 1 bis 4, wobei der Roboter (30) konfiguriert ist, um im Betrieb eine Schraube in das Pedikel eines Wirbels des Patienten einzuführen, der auf dem Operationstisch (10) liegt.

7. Installation nach einem der vorhergehenden Ansprüche, wobei der Roboter (30) konfiguriert ist, um im Betrieb einen chirurgischen Eingriff zu unterstützen, der von einem Chirurgen am Patienten (P) vorgenommen wird, der auf dem Operationstisch (10) liegt, insbesondere um ein Bohrwerkzeug zu führen, das vom Chirurgen auf das Pedikel eines Wirbels des Patienten angewendet wird.

8. Installation nach einem der vorhergehenden Ansprüche, wobei die Säule (21, 22) mit Elementen zur Verschiebung und Stabilisierung (24) mit Bezug auf den Boden versehen sind, wobei diese Elemente zur Verschiebung und Stabilisierung im Betrieb ausgelegt sind, um alternativ die Position der Tischbrücke (20) entlang des Patienten (P), der auf dem Operationstisch (10) liegt, durch Verschieben auf dem Boden und Blockieren der Tischbrücke auf stabile Weise auf dem Boden zu ändern.

9. Installation nach Anspruch 8, wobei die Elemente zum Verschieben und Stabilisieren Rollen mit Bremsvorrichtung (24) sind.

10. Installation nach einem der vorhergehenden Ansprüche, wobei eine der Säulen (21, 22) einen Schrank (25) einschließt, in dessen Innerem eine elektronische Einheit (40) aufgenommen ist, die ausgelegt ist, um den Roboter (30) zu steuern.

11. Installation nach einem der vorhergehenden Ansprüche, wobei die Tischbrücke (20) auf einer oberen Fläche (23A) ihrer Plattform (23) mindestens einen Bereich zur Bereitstellung (26) der chirurgischen Instrumente aufweist.

12. Installation nach einem der vorhergehenden Ansprüche, wobei die Tischbrücke (20) in mindestens einer ihrer Säulen (11, 12) mindestens einen Bereich zur Bereitstellung (26) der chirurgischen Instrumente aufweist.

## Claims

1. An installation (1) for robotic spine surgery, comprising:
- an operating table (10), which is designed to lay a patient (P) thereon,
- a robot (30) for posterior spine surgery, wherein the robot (30) is configured, during use, to perform a surgical procedure on the patient (P) recumbent on the operating table (10), and
- a bridge table (20) comprising:
- two uprights (21, 22) that are designed to be separated from the operating table when the installation is in use, while being arranged and rising from the ground on either side of the patient recumbent on the operating table, and
- a platform (23) to support the robot (30), which rests on the uprights so that, during use, the platform lies over the patient recumbent on the operating table and which is designed, during use, to support the robot extending vertically above the spine (R) of the patient recumbent on the operating table
**characterized in that** wherein the platform (23) incorporates a presentation area (27) for spinal implants, said presentation area being covered by a field of activity over which the robot (30) is arranged to extend.

2. The installation according to claim 1, wherein the robot (30) is designed to extend over a field of activity that, when the installation (1) is in use and the bridge table (20) occupies a fixed position alongside the patient (P) recumbent on the operating table (10), covers several vertebrae (V) of the patient, in particular between three and eight vertebrae of the patient.

3. The installation according to any of the preceding claims, wherein the robot (30) comprises:
- a foot (31) that is attached to the platform (23) of the bridge table (20) so as, during use, to be substantially centered on the sagittal plane (PS) of the patient (P) recumbent on the operating table (10), and
- a robotic arm (32) that extends from the foot (31), being positioned, during use, plumb with the spine (R) of the patient (P), and which terminates in a spinal surgery effector (33).

4. The installation according to claim 3, wherein the bridge table (2) is designed, during use, to position the foot (31) of the robot (30) vertically above the pelvic girdle (CP) of the patient (P) recumbent on the operating table (10).

5. The installation according to any one of the preceding claims, wherein the robot (30) is configured, during use, to pierce the pedicle of a vertebra (V) of the patient recumbent on the operating table (10).

6. The installation according to any one of claims 1 to 4, wherein the robot (30) is configured, during use, to insert a screw into the pedicle of a vertebra of the patient recumbent on the operating table (10).

7. The installation according to any one of the preceding claims, wherein the robot (30) is configured, during use, to assist a surgical act applied by a surgeon to the patient (P) recumbent on the operating table (10), in particular to guide a drilling tool applied by the surgeon on the pedicle of a vertebra of the patient.

8. The installation according to any one of the preceding claims, wherein the uprights (21, 22) are provided with movement and stabilization elements (24) in relation to the ground, these movement and stabilization elements, during use, being designed to alternatively alter the position of the bridge table (20) along the patient (P) recumbent on the operating table (10) by movement on the ground and to immobilize the bridge table in a stable manner on the ground.

9. The installation according to claim 8, wherein the movement and stabilization elements are casters with brakes (24).

10. The installation according to any one of the preceding claims, wherein one of the uprights (21, 22) incorporates a cabinet (25) inside which an electronic unit (40) is housed, designed to control the robot (30).

11. The installation according to any one of the preceding claims, wherein the bridge table (20), on an upper face (23A) of its platform (23), integrates at least one surgical instrumentation provisioning area (26).

12. The installation according to any one of the preceding claims, wherein the bridge table (20) integrates at least one surgical instrumentation provisioning area (26) in at least one of its uprights (11, 12).
